(19) Europäisches Patentamt
European Patent Office
Office européen des brevets

(11) Numéro de publication: **0 043 756 B1**

(12) **FASCICULE DE BREVET EUROPEEN**

(45) Date de publication du fascicule du brevet: **09.05.84**

(51) Int. Cl.³: **C 07 D 501/36**, A 61 K 31/545

(21) Numéro de dépôt: **81401028.6**

(22) Date de dépôt: **26.06.81**

(54) **1,2,4-Triazinylthiométhyl-3-céphèmes sulfoxydes, procédé de préparation et compositions pharmaceutiques en contenant.**

(30) Priorité: **30.06.80 FR 8014512**
**03.03.81 FR 8104242**
**03.03.81 FR 8104243**

(43) Date de publication de la demande:
**13.01.82 Bulletin 82/2**

(45) Mention de la délivrance du brevet:
**09.05.84 Bulletin 84/19**

(84) Etats contractants désignés:
**AT BE CH DE FR GB IT LI LU NL SE**

(56) Documents cités:
**EP - A - 0 002 605**
**FR - A - 2 387 234**
**GB - A - 2 049 675**

***Le dossier contient des informations techniques présentées postérieurement au dépôt de la demande et ne figurant pas dans le présent fascicule.***

(73) Titulaire: **SANOFI, société anonyme, 40, Avenue George V, F-75008 Paris (FR)**

(72) Inventeur: **Labeeuw, Bernard, 49 Lotissement des Genêts avenue des Moulins, F-34000 Montpellier (FR)**
Inventeur: **Salhi, Ali, 639 rue Valène, F-34980 St. Gely du Fesc (FR)**

(74) Mandataire: ***Combe, André et al,* CABINET BEAU DE LOMENIE 55 rue d'Amsterdam, F-75008 Paris (FR)**

**EP 0 043 756 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux sulfoxydes de céphalosporines, un procédé pour leur préparation et des compositions pharmaceutiques renfermant lesdits sulfoxydes de céphalosporines en tant qu'ingrédients actifs.

Plus particulièrement, la présente invention se réfère à de nouvelles céphalosporines sulfoxydes ayant en position 3 du noyau céphème un groupement 1,3,4-triazinylthiométhyle.

Le brevet belge N° 866038 décrit une série de sulfoxydes et de sulfones de céphalosporines ayant la formule générale suivante:

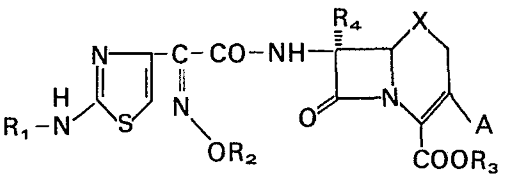

(I)

Le brevet ci-dessus cite, parmi les radicaux indiqués pour A, des groupes $CH_2SR_5$ où $R_5$ peut être triazinyle, en particulier 1,3,4-triazin-2-yle et 1,3,5-triazin-1-yle éventuellement substitués et, plus particulièrement, des composés de formule (I) ci-dessus où:

$R_1 = H$; $R_2 = H$; $R_3 = H$; $R_4 = H$; X = SO

A = 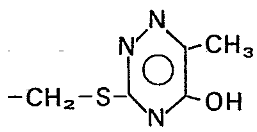

(II)

$R_1 = H$; $R_2 = CH_2-CH=CH_2$;

$R_3$ = 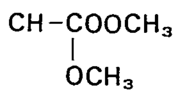

$R_4 = H$; X = SO

A = 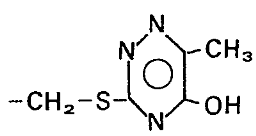

(III)

sans toutefois les décrire d'une façon spécifique.

Les céphalosporines de formule I ci-dessus sont censées posséder d'une façon générale une très forte activité antibactérienne contre les bactéries Gram positives et Gram négatives et être très efficaces contre les staphylocoques formant de la pénicillinase et montrent également en partie une activité fongistatique.

On a maintenant trouvé que certaines céphalosporines 1-oxyde ayant en position 3 du noyau céphème un groupe 1,2,4-triazin-3-yle substitué en position 5 par un hydroxyle ou un groupe 1,2,4-triazin-5-yle substitué en position 3 par un hydroxyle montrent un profil antibactérien complètement différent de celui des composés décrits dans le brevet belge N° 866038.

On a trouvé d'une façon surprenante que lesdites céphalosporines 1-oxyde ont une activité remarquable sur les bactéries Gram négatives, y compris celles productrices de β-lactamases et, en particulier, les entérobactéries, tandis qu'elles sont pratiquement inactives sur les staphylocoques producteurs ou non de β-lactamases.

Ainsi, la présente invention a pour objet de nouvelles (3-hydroxy-1,2,4-triazinyl)thiométhylcéphalosporines de formule:

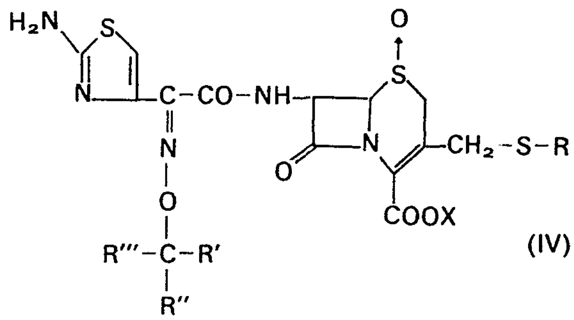

(IV)

dans laquelle:

— R représente un groupe 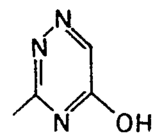

ou un groupe 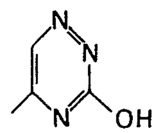

R' représente de l'hydrogène ou un groupe carboxyle COOX',

R'' et R''', qui peuvent être identiques ou différents, représentent chacun de l'hydrogène ou un groupe alkyle inférieur ou, ensemble, un radical 1,3-propylène ou 1,4-butylène,

X et X', qui peuvent être identiques ou différents, représentent de l'hydrogène ou un cation ou un ester ou hémiacétal facilement hydrolysable ou métaboliquement labile et pharmaceutiquement acceptable.

Le terme alkyle inférieur, tel qu'utilisé ici, désigne le radical d'un hydrocarbure aliphatique saturé contenant jusqu'à 3 atomes de carbone, à savoir méthyle, éthyle, propyle et isopropyle.

Le terme cation, tel qu'utilisé ici, désigne un ion alcalin ou alcalino-terreux, de préférence les ions sodium, potassium ou calcium, ou bien le proton lié à une amine organique pharmaceutiquement acceptable telle que l'éthylènediamine, la triéthanolamine, la diéthanolamine, l'éthanolamine, la thrométamine et similaires pour former des sels d'addition.

Le terme ester ou hémiacétal facilement hydrolysable ou métaboliquement labile et pharmaceutiquement acceptable désigne les radicaux qui forment en général des produits avec des acides carboxyliques pharmacologiquement actifs, de préférence les radicaux phtalidyle, pivaloyloxyméthyle, acétoxyméthyle, éthoxycarbonyloxyméthyle, 1-(éthoxycarbonyloxy)éthyle, acétonyle, α-méthoxy-α-carbométhoxyméthyle, carbométhoxyméthyle, carbéthoxyméthyle et similaires.

Par suite de la présence dans la formule d'un groupement oxime, les composés (IV) existent sous deux formes isomères, syn et anti. Les isomères syn dont l'activité thérapeutique est supérieure sont les composés préférés.

Il est entendu que les composés (IV) indiqués ci-dessus peuvent exister:

— soit sous la forme indiquée dans la formule (IV),

— soit sous la forme tautomère (IV'):

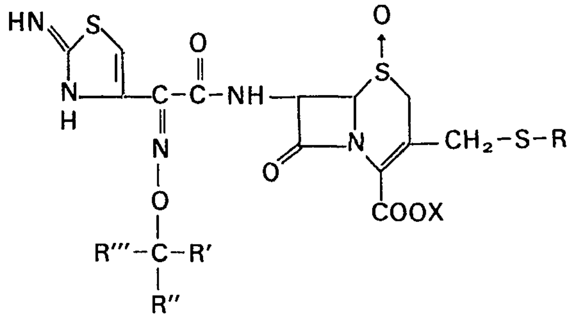

dans laquelle R, R', R'', R''' et X ont les significations indiquées précédemment.

L'invention concerne également des procédés de préparation des composés de formule (IV).

Un premier procédé comprend les étapes schématisées ci-après.

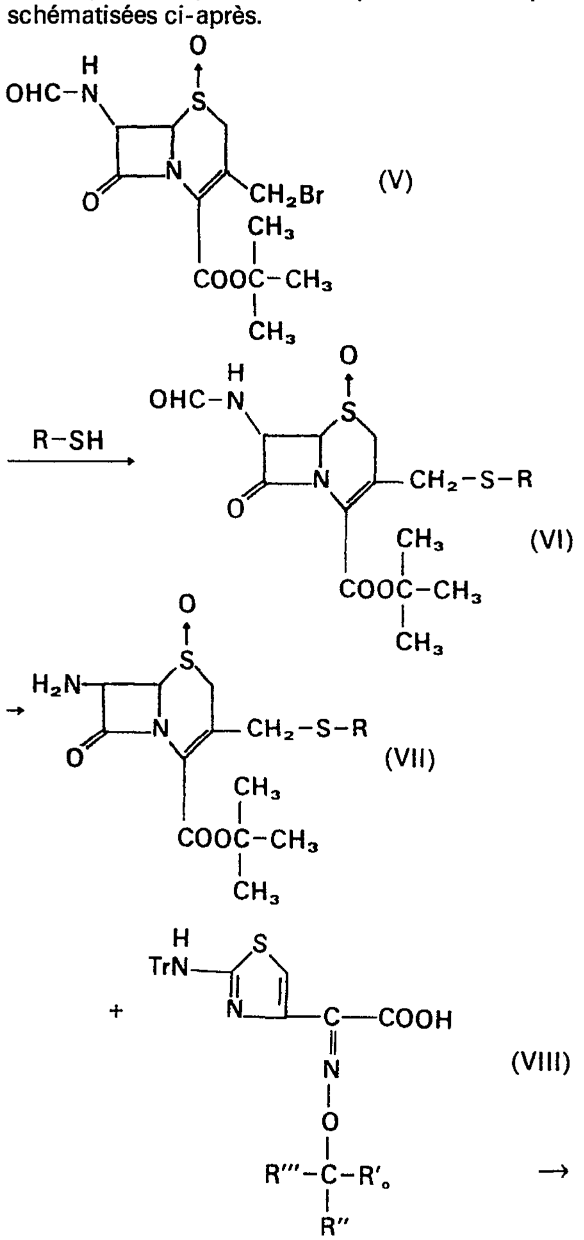


(V)

(VI)

(VII)

(VIII)

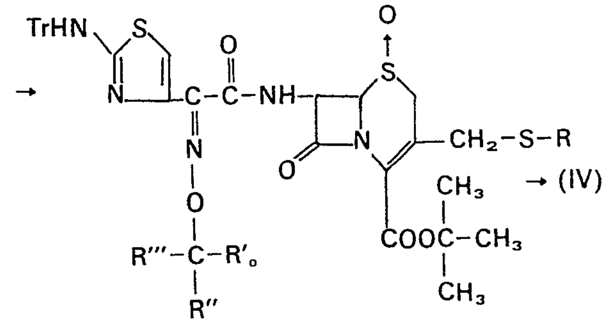

(IX)

Tr = trityle.

On part du sulfoxyde (V) qui, par action du composé R−SH en solution dans le diméthylformamide en présence d'une base comme la triéthylamine, conduit au composé convenablement substitué en position 3 (VI). On libère le groupe amino de celui-ci par action du chlorure de thionyle en solution et on isole le composé (VII) sous forme de chlorhydrate. Celui-ci est acylé par le composé de formule (VIII) dans laquelle R'' et R''' ont la signification donnée ci-dessus et $R'_o$ représente de l'hydrogène ou un groupe $COOX'_o$ où $X'_o$ représente un groupe hémiacétal ou ester facilement hydrolysable, t-butyle de préférence.

Avant d'effectuer la réaction d'acylation, il est souhaitable de substituer le groupe amino de l'acide par un groupe protecteur facile à éliminer ultérieurement. On peut utiliser les groupes habituellement utilisés en synthèse organique pour la protection des groupes aminés et, en particulier, le groupe trityle.

Pour effectuer la réaction d'acylation, il est nécessaire de procéder à l'activation du groupe carboxyle du composé (VIII), de préférence par transformation en anhydride à l'aide d'un carbodiimide, en général le dicyclohexylcarbodiimide.

La réaction d'activation est effectuée au sein d'un solvant organique convenable tel que le tétrahydrofuranne à une température comprise entre 0 et 50° C et, de préférence, à température ambiante. La réaction d'activation est éventuellement facilitée par addition d'un dérivé hydroxylé tel que l'hydroxy-1 benzotriazole.

La solution du réactif d'acylation ainsi obtenue, débarrassée par filtration de la dicyclohexylurée formée, est ajoutée à une solution du composé (VII) dans un solvant tel que le diméthylformamide. L'addition des deux réactifs peut aussi s'effectuer dans l'ordre inverse.

Après la réaction d'acylation, le groupe protecteur sur l'amine, l'ester tertiobutylique et le groupement $X'_o$ sont éliminés par un procédé connu, en particulier par hydrolyse en milieu acide en utilisant un acide organique tel que l'acide formique ou l'acide trifluoroacétique.

En ce qui concerne les matières premières de la réaction, les composés (V) et le composé (VIII) ainsi que ses dérivés dans lesquels le groupe aminé est bloqué par un groupe protecteur sont connus.

Une variante de ce procédé consiste à acyler tout d'abord l'amino-7 bromométhyl-3 céphème-3 carboxylate de tertiobutyle-4 (X) par l'acide (VIII).

On opère ainsi qu'il est indiqué pour l'acylation des composés (VII).

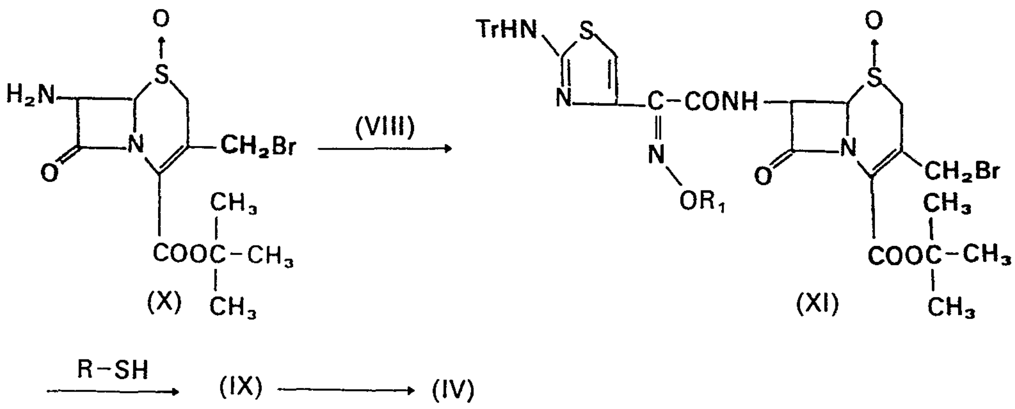


(X) (XI) (IX) (IV)

Par action sur le composé (XI) ainsi obtenu du thiol R-SH, on obtient le composé (IX) correspondant. On opère dans les conditions indiquées pour l'obtention des composés (VI).

Comme indiqué précédemment, les composés (IX) traités par un acide fort conduisent aux composés (IV).

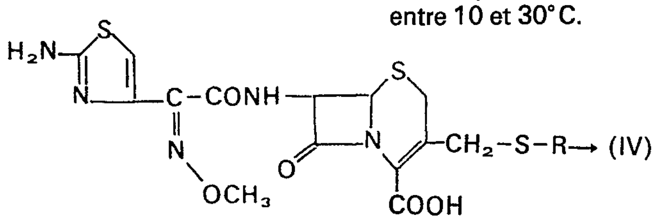

(XII)

Un second procédé de préparation permet d'obtenir les composés (IV) à partir du dérivé correspondant non sulfoxydé par action d'un peracide organique tel que l'acide chloro-3 perbenzoïque.

On opère en solution à température comprise entre 10 et 30°C.

Les composés (IV) de l'invention, dans lesquels X est autre que H, s'obtiennent à partir des composés (IV) dans lesquels X est H par des réactions connues en elles-mêmes.

Ainsi, les sels minéraux sont obtenus par action sur les composés (IV) dans lesquels X est H d'une base minérale telle que la soude ou la potasse ou le bicarbonate de sodium, en quantité équimoléculaire; la réaction de salification est réalisée dans un solvant tel que l'eau ou l'éthanol, et le sel obtenu est isolé par évaporation de la solution.

Les sels de bases organiques sont obtenus par action, sur une solution de l'acide (IV, X=H), dans un solvant ou un mélange de solvants convenable, d'une quantité équimoléculaire de la base organique. Le sel est isolé par précipitation avec l'éther.

On peut également préparer les sels des composés (IV) dans lesquels R'=COOX', où X' est H ainsi que, de préférence, les sels obtenus par salification des composés (IV) dans lesquels X est H et R' est COOX' où X' est H.

Les hémiacétals et les esters sont obtenus par les procédés connus d'estérification; par exemple, on utilisera avantageusement l'action d'un dérivé halogéné sur un sel tel que le sel de sodium de l'acide; on réalisera de préférence la réaction dans un solvant capable de dissoudre le dérivé acide de départ, par exemple dans le diméthylformamide.

On peut ainsi préparer les dérivés des composés de formule (IV) où un des X est X' et autre que l'hydrogène ou dans lesquels tous les deux sont autres que de l'hydrogène.

Les isomères de forme syn et anti s'obtiennent par un choix convenable des réactifs.

Les nouveaux composés de la présente invention possèdent une activité remarquable sur les bactéries Gramm négatives, et notamment sur les souches productrices de β-lactamases.

En particulier, les composés de formule IV ci-dessus sont très actifs aussi bien sur *Pseudomonas* que sur *Enterobacter*, ce qui est très particulièrement surprenant pour des céphalosporines. Ils sont également actifs sur certaines souches de *Proteus*, de *Serratia* et de *Klebsiella*.

Les composés de la présente invention ont aussi une durée d'action particulièrement élevée; par exemple, l'acide 7-[2-(2-amino-4-thiazolyl)-2-(1-carboxy-1-méthyléthoxy)imino-acétamido]-3-(5-hydroxy-triazin-2-ylthiométhyl)-3-céphème-4-carboxylique 1-S-oxyde isomère syn désigné par son numéro de code CM 40766 possède une demi-vie de plus de 4 h.

Les composés de la présente invention sont très peu toxiques.

La présente invention, selon un autre de ses aspects, se réfère ainsi à des compositions pharmaceutiques renfermant, en tant qu'ingrédients actifs, les composés de formule (IV) ci-dessus.

Les composés de la présente invention sont très peu toxiques.

La présente invention, selon un autre de ses aspects, se réfère ainsi à des compositions

pharmaceutiques renfermant, en tant qu'ingrédients actifs, les composés de formule (IV) ci-dessus.

Les compositions pharmaceutiques de la présente invention peuvent être formulées pour l'administration orale, sublinguale, sous-cutanée, intramusculaire, intraveineuse, transdermique ou rectale, en mélangeant les ingrédients actifs avec des supports pharmaceutiques classiques. Elles peuvent être administrées sous des formes unitaires d'administration aux animaux et aux êtres humains dans le traitement des infections dues en particulier à des germes Gram négatifs, y compris les producteurs de β-lactamases.

Afin d'obtenir l'effet antibactérien désiré, la dose de principe actif peut varier entre 10 et 500 mg/kg de poids par jour.

Chaque dose unitaire peut contenir de 10 à 10 000 mg d'ingrédient actif en combinaison avec un support pharmaceutique. Cette dose unitaire peut être administrée 1 à 4 fois par jour. Les formes unitaires d'administration comprennent les comprimés, les gélules, les poudres, les granulés et les solutions ou suspensions orales et les tablettes pour l'administration sublinguale, les suppositoires ainsi que les ampoules utiles pour une administration parentérale.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de sucrose ou d'autres matières appropriées ou encore on peut les traiter de telle sorte qu'ils aient une activité prolongée ou retardée et qu'ils libèrent continuellement une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant acalorique, du méthylparaben et du propylparaben comme antiseptiques, ainsi qu'un agent donnant du goût et un colorant approprié.

Les poudres ou les granulats dispersibles dans l'eau peuvent contenir l'ingrédient actif en mélange avec des agents de dispersion ou des agents mouillants, ou des agents de mise en suspension, tels que la polyvinylpyrrolidone et similaires, de même qu'avec des édulcorants ou des correcteurs du goût.

Pour une administration orale en gouttes ou pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les exemples suivants permettent de mieux comprendre la portée de l'invention.

Ainsi qu'il est habituel dans cette famille de composés, les produits suivant l'invention ne présentent pas de point de fusion net, mais seulement des points de décomposition ne permettant pas de les caractériser.

Les produits seront donc caractérisés par leur spectre de résonance magnétique nucléaire enregistré à 60 MHz, l'étalon interne étant l'hexaméthyldisiloxanne.

Les abréviations suivantes seront utilisées:

— S: singulet
— D: doublet
— D de D: doublet de doublet
— S. e.: singulet élargi
— M: multiplet
— AB: système AB
— J: représente la constante de couplage.

De plus, les microanalyses élémentaires ont été effectuées dans chaque cas et sont en accord avec les formules indiquées.

*Exemple 1:*

*Acide [(amino-2 thiazolyl-4)-2 méthoxyimino-2 acétamido]-7 [(hydroxy-3 triazin-1,2,4 yl-5) thiométhyl]-3 céphème-3 carboxylique-4 S-oxyde-1 isomère syn.*

(CM 40 583)

*a) [(Tritylamino-2 thiazolyl-4)-2 méthoxyimino-2 acétamido]-7 bromométhyl-3 céphème-3 carboxylate de t-butyle-4 S-oxyde-1 isomère syn.*

A une solution de 3,26 g de chlorhydrate d'amino-7 bromoéthyl-3 céphème-3 carboxylate de tertiobutyle-4 S-oxyde-1 dans 58 ml de chlorure de méthylène anhydre, on ajoute 1,12 ml de triéthylamine, 3,94 g d'acide (tritylamino-2 thiazolyl-4)-2 t-butoxycarbonylméthoxyimino-2 acétique isomère syn, 1,84 g de dicyclohexylcarbodiimide et 0,1 g d'hydroxy-1 benzotriazole.

On agite à température ambiante pendant 3½ h, puis on filtre la dicyclohexylurée formée. On concentre le solvant à environ 10 ml sous vide, puis on chromatographie sur une colonne de gel de silice.

Par élution avec un mélange hexane/acétate d'éthyle 60/40 (vol/vol), on obtient le produit attendu (2,4 g).

*Spectre de RMN*

1 H à 8,82 ppm (NH−CO, D, J=8 Hz) - 1 H à 8,70 ppm (NH−trityle, S) - 15 H à 7,32 ppm (H aromatique, S) - 1 H à 6,78 ppm (H thiazole, S) - 1 H à 5,79 ppm ($H_7$, D de D, $J_1$=8 Hz, $J_2$=4,5 Hz) - 1 H à 4,96 ppm ($H_6$, D, J=4,5 Hz) - 2 H à 4,50 ppm ($CH_2$ Br, S. e.) - 3 H à 3,78 ppm ($CH_3O$−, S) - 2 H à 3,77 ppm ($CH_2S$→O, S. e.) - 9 H à 1,46 ppm

$CH_3$
|
(COOC−$CH_3$, S).
|
$CH_3$

*b) [(Tritylamino-2 thiazolyl-4)-2 méthoxyimino-2 acétamido]-7 [(hydroxy-3 triazin-1,2,4 yl-5 thiométhyl]-3 céphème-3 carboxylate de t-butyle S-oxyde-1 isomère syn.*

On agite pendant 2 h à température ambiante la solution de 0,8 g du dérivé bromé de l'exemple 1 a) et 0,16 g d'hydroxy-3 mercapto-5 triazine-1,2,4 dans le diméthylformamide.

On évapore le solvant sous vide et reprend le résidu dans l'éther isopropylique. On essore le précipité et on le redissout dans le minimum de chlorure de méthylène. On chromatographie sur une colonne de gel de silice (50 g).

En éluant avec de l'acétate d'éthyle, on obtient 0,55 g du produit attendu.

*c) CM 40 583.*

On laisse pendant 30 min à température ambiante la solution de 0,310 g du produit obtenu au paragraphe b) dans 3,1 ml d'acide trifluoracétique. On concentre sous vide à 2 ml et précipite le produit par addition d'éther isopropylique. On essore le solide et sèche sous vide.

Le solide est purifié par agitation dans 66 ml d'éthanol à 90° qui fournit 0,100 g de CM 40 583. Par traitement sur charbon de la solution et concentration à petit volume, on obtient encore 0,030 g du produit attendu.

*Spectre de RMN*

1 H à 8,95 ppm (N$\underline{H}$−CO−, D, J=8,5 Hz) - 1 H à 7,98 ppm (H triazine, S) - 4 H à 7,00 ppm (O$\underline{H}$, COO$\underline{H}$, N$\underline{H}_2$, S.e.) - 1 H à 6,89 ppm (H thiazole, S) - 1 H à 5,88 ppm ($H_7$, D de D, $J_1$=9 Hz, $J_2$=4 Hz) - 1 H à 4,96 ppm ($H_6$, D, J=4 Hz) - 1 H à 4,42 ppm (C$\underline{H}$S, A de AB, J=13 Hz) - 1 H à 4,05 ppm (C$\underline{H}$S, B de AB, J=13 Hz) - 5 H à 3,85 ppm (C$\underline{H}_3$O− et C$\underline{H}_2$S→O, M).

*Exemple 2:*

*Acide [(amino-2 thiazolyl-4)-2 carboxyméthoxyimino-2 acétamido]-7 (hydroxy-3 triazin-1,2,4 yl-5) thiométhyl]-3 céphème-3 carboxylique 4 S-oxyde-1 isomère syn.*

(CM 40 515)

*a) [(Tritylamino-2 thiazolyl-4)-2 t-butoxycarbonyl-méthoxyimino-2 acétamido]-7 bromométhyl-3 céphème-3 carboxylate de tertiobutyle-4 S-oxyde-1 isomère syn.*

A une solution de 3,26 g de chlorhydrate d'amino-7 bromométhyl-3 céphème-3 carboxylate de tertiobutyle-4 S-oxyde-1 dans 58 ml de chlorure de méthylène anhydre, on ajoute 1,12 ml de triéthylamine, 4,86 g d'acide (tritylamino-2 thiazolyl-4)-2 t-butoxycarbonyl méthoxyimino-2 acétique isomère syn, 1,84 g de dicyclohexylcarbodiimide et 0,1 g d'hydroxy-1 benzotriazole.

On agite à température ambiante pendant 3½ h, puis on filtre la dicyclohexylurée formée. On concentre le solvant à environ 10 ml sous vide, puis on chromatographie sur une colonne de gel de silice.

Par élution avec un mélange hexane-acétate d'éthyle 60-40 (vol/vol), on obtient le produit attendu (2,8 g).

*Spectre de RMN*

1 H à 8,75 ppm (N$\underline{H}$-Trit, S) - 1 H à 8,57 ppm (N$\underline{H}$−CO, D, J=8,5 Hz) - 15 H à 7,28 ppm (H aromatiques, S) - 1 H à 6,82 ppm (H thiazole, S) - 1 H à 5,84 ppm ($H_7$, D de D, $J_1$=8,5 Hz, $J_2$=4,5 Hz) - 1 H à 4,98 ppm ($H_6$, D, J=4,5 Hz) - 4 H à 4,50 ppm (C$\underline{H}_2$Br et OC$\underline{H}_2$, S) - 2 H à 3,72 ppm (C$\underline{H}_2$S→O, S. e.) - 9 H à 1,44 ppm (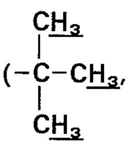, S) - 9 H à 1,35 ppm (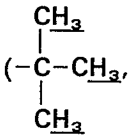, S).

*b) [(Tritylamino-2 thiazolyl-4)-2 t-butoxycarbonyl méthoxyimino-2 acétamido]-7 [(hydroxy-3 triazin-1,2,4 yl-5)thiométhyl]-3 céphème-3 carboxylate de tertiobutyle-4 S-oxyde-1 isomère syn.*

On agite pendant 2 h à température ambiante la solution de 0,8 g du dérivé bromé de l'exemple 2a) et 0,16 g d'hydroxy-3 mercapto-5 triazine-1,2,4 dans le diméthylformamide.

On évapore le solvant sous vide et reprend le résidu dans l'éther isopropylique. On essore le précipité et on le redissout dans le minimum de chlorure de méthylène. On chromatographie sur une colonne de gel de silice (50 g).

En éluant avec de l'acétate d'éthyle, on obtient 0,57 g du produit attendu.

*c) CM 40 515*

On opère sur 0,2 g du produit ci-dessus ainsi qu'il est indiqué dans l'exemple 1c).

On obtient 0,15 g de CM 40 515.

*Spectre de RMN*

1 H à 8,74 ppm (N$\underline{H}$ CO, D, J=8,5 Hz) - 1 H à 7,97 ppm (H triazine, S) - 1 H à 6,93 ppm (H thiazole, S) - 5H à 6,50 ppm (H échangeables, S. e.) - 1 H à 5,95 ppm ($H_7$, D de D $J_1$=8,5 Hz, $J_2$=4,5 Hz) - 1 H à 4,95 ppm ($H_6$, D, J=4,5 Hz) - 2 H à 4,59 ppm (OC$\underline{H}_2$, S) - 1 H à 4,40 ppm (C$\underline{H}$ S-triazine, A de AB, $J_{AB}$=15 Hz) - 1 H à 4,0 ppm (C$\underline{H}$ S triazine, B de AB, $J_{AB}$=15 Hz) - 2 H à 3,75 ppm (C$\underline{H}_2$ S→O, S. e.).

*Exemple 3:*

*Acide [(amino-2 thiazolyl-4)-2 (carboxy-2 propyl-2 oxymino)-2 acétamido]-7 [(hydroxy-3 triazin-1,2,4 yl)-5 thiométhyl]-3 céphème-3 carboxylique-4 S-oxyde-1 isomère syn.*

(CM 40 421)

*a) [(Tritylamino-2 thiazolyl-4)-2 (t-butoxycarbonyl-2 propyl-2 oximino)-2 acétamido]-7 bromométhyl-3 céphème-3 carboxylate de tertiobutyle-4 S-oxyde-1 isomère syn.*

A une solution de 5 g de chlorhydrate d'amino-7

bromométhyl-5 céphème 3 carboxylate de tertiobutyle-4 S-oxyde-1 dans 90 ml de chlorure de méthylène, on ajoute 1,72 ml de triéthylamine, 7,57 g d'acide (tritylamino-2 thiazolyl-4)-2 (t-butoxycarbonyl-2 propyl-2 oximino)-2 acétique, 2,84 g de dicyclohexylcarbodiimide et 0,1 g d'hydroxy benzotriazole. On agite le mélange pendant 15 h à température ambiante, puis on filtre la dicyclohexylurée formée.

Après évaporation du solvant, on chromatographie le résidu sur une colonne de gel de silice (250 g). En éluant avec un mélange hexane-acétate d'éthyle 50-50 (vol/vol), on obtient 4,3 g du produit attendu.

*Spectre de RMN*

(en solution dans le diméthylsulfoxyde deutérié).

1 H à 8,70 ppm (NH−Trit, S) - 1 H à 8,07 ppm (NH−CO, D, J=9 Hz) - 15 H à 7,25 ppm (H−Trit, S) - 1 H à 6,72 ppm (H thiazole, S) - 1 H à 5,88 ppm ($H_7$, D de D, $J_1$=9 Hz, $J_2$=4 Hz) - 1 H à 4,96 ppm ($H_6$, D, J=4 Hz) - 2 H à 4,50 ppm ($CH_2$Br, AB, $J_{AB}$=12 Hz) - 2 H à 3,77 ppm ($CH_2$ en 2, S. el) - 9 H à 1,45 ppm (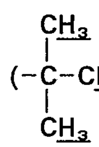, S) - 6 H à 1,37 ppm (−C− , S) - 9 H à 1,27 ppm (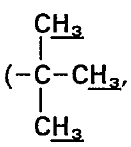, S).

*b) [(Tritylamino-2 thiazolyl-4)-2 (t-butoxycarbonyl-2 propyl-3 oximino)-2 acétamido]-7 [(hydroxy-3 triazin-1,2,4 yl)-5 thiométhyl]-3 céphème-3 carboxylate de tertiobutyle-4 S-oxyde-1 isomère syn.*

A une solution de 1 g du produit obtenu précédemment dans 12 ml de diméthylformamide, on ajoute 0,18 g d'hydroxy-3 mercapto-5 triazine-1,2,4, puis 0,15 ml de triéthylamine. On agite 3 h à température ambiante, puis on précipite le produit attendu par addition d'éther isopropylique.

*c) CM 40 421*

On agite pendant 30 min à température ambiante une solution de 0,89 g du composé obtenu ci-dessus dans 9 ml d'acide trifluoracétique. On concentre la solution sous vide jusqu'à un volume de 5 ml et ajoute de l'éther isopropylique jusqu'à précipitation. On essore le précipité et on le redissout dans 800 ml d'un mélange acétone-éthanol 50-50 (vol/vol). On concentre sous vide jusqu'à 50 ml puis on essore le précipité.

*Spectre de RMN*

(en solution dans le diméthylsulfoxyde deutérié).

1 H à 8,35 ppm (NHCO, D, J=9 Hz) - 1 H à 7,95 ppm ($H_6$ triazine, S) - 5 H à 7,30 ppm (2 $CO_2H$, $NH_2$, OH, S. el) - 1 H à 6,85 ppm (H thiazole, S) - 1 H à 5,96 ppm (D de D, $J_1$= 9 Hz, $J_2$=4 Hz) - 1 H à 4,95 ppm ($H_6$, D, J=4 Hz) - 2 H à 4,20 ppm ($CH_2S$, AB, $J_{AB}$=13 Hz) - 2 H à 3,75 ppm ($CH_2$ en 2, S. el) - 6 H à 1,43 ppm ($CH_3$, S).

*Exemple 4:*

*Acide [(amino-2 thiazolyl-4)-2 (carboxy-1 cyclobutyl-2 oxyimino)-2 acétamido]-7 [(hydroxy-3 triazin-1,2,4 yl-5) thiométhyl]-3 céphème-3 carboxylique-4 S-oxyde-1 isomère syn.*

(CM 40 732)

*a) [(Tritylamino-2 thiazolyl-4)-2 (t-butoxycarbonyl-1 cyclobutyl-1 oxyimino)-2 acétamido]-7 bromométhyl-3 céphème-3 carboxylate de t-butyle-4 S-oxyde-1 isomère syn.*

A une solution de 4,4 g de chlorhydrate d'amino-7 bromométhyl-3 céphème-3 carboxylate de t-butyle-4 S-oxyde-1 dans 70 ml de chlorure de méthylène anhydre, on ajoute, sous atmosphère d'azote, 1,5 ml de triéthylamine, 5,1 g d'acide (tritylamino-2 thiazolyl-4)-2 (t-butoxycarbonyl-1 cyclobutyl-1 oxyimino)-2 acétique isomère syn, 2,4 g de dicyclohexylcarbodiimide et 0,1 g d'hydroxy-1 benzotriazole. On agite durant 1 h à température ambiante, puis on filtre la dicyclohexylurée formée et concentre la solution à 20 ml sous vide. On chromatographie sur une colonne de gel de silice (150 g). Par élution avec le mélange hexane-acétate d'éthyle 40-60 (vol/vol), on obtient, après évaporation du solvant, 4,8 g du produit attendu.

*Spectre de RMN*

1 H à 7,90 ppm (NHCO, D, J=9 Hz) - 15 H à 7,26 ppm (H aromatiques, S) - 1 H à 6,97 ppm (NH−trityle, S. e.) - 1 H à 6,65 ppm (H thiazole, S) - 1 H à 6,18 ppm ($H_7$, D de D, $J_1$=9 Hz, $J_2$= 4,5 Hz) - 2 H à 3,4 ppm ($CH_2S$→O, S. e.) - 6 H entre 1,5 et 2,6 ppm (cyclobutyle, M) - 9 H à 1,46 ppm (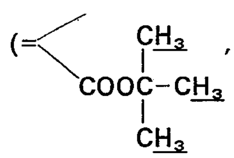, S) - 9 H à 1,36 ppm (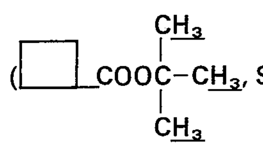, S).

b) et c)

A partir du dérivé trouvé ci-dessus et en opérant comme dans l'exemple 3b) avec l'hydroxy-3 mercapto-5 triazine-1,2,4 et après déblocate des fonctions amines et acides selon l'exemple 3c), on obtient le produit attendu.

*Spectre de RMN*

1 H à 13 ppm (S. e., OH) - 5 H entre 8 et 10 ppm (M, CONH, COOH, $NH_2$) - 1 H à 7,95 ppm (S, H triazine) - 1 H à 6,83 ppm (S, H thiazole) - 1 H à

6,00 ppm (M, $H_7$) - 1 H à 4,97 ppm (M, $H_6$) - 1 H à 4,40 ppm (A de AB, $J_{AB}$=13 Hz, $C\underline{H_2}S$) - 1 H à 4,00 ppm (B de AB, $J_{AB}$=13 Hz, $C\underline{H_2}S$) - 2 H à 3,97 ppm (M, $C\underline{H_2}S\rightarrow O$) - 6 H entre 1,5 et 2,5 ppm (M, 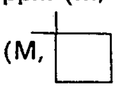 )

*Exemple 5:*

*Acide [(amino-2 thiazolyl-4)-2 (carboxy-1 cyclopentyl-1 oxyimino)-2 acétamido]-7 [(hydroxy-3 triazin-1,2,4 yl-5)thiométhyl]-3 céphème-3 carboxylique-4 S-oxyde-1 isomère syn.*

*a) [(Tritylamino-2 thiazolyl-4)-2 (t-butoxycarbonyl-1 cyclopentyl-1 oxyimino)-2 acétamido]-7 bromométhyl-3 céphème-3 carboxylate de t-butyle-4 S-oxyde-1 isomère syn.*

On opère comme dans l'exemple 4 a) en utilisant l'acide (tritylamino-2 thiazolyl-4)-2 (t-butoxycarbonyl-1 cyclopentyl-1 oxyimino)-2 acétique syn.

*Spectre de RMN*

1 H à 7,83 ppm ($N\underline{H}CO$, D, J=9 Hz) - 15 H à 7,27 ppm (H aromatiques, S) - 1 H à 6,93 ppm ($N\underline{H}$-trityle, S. e.) - 1 H à 6,14 ppm ($H_6$, D de D, $J_1$=9 Hz, $J_2$=4,5 Hz) - 2 H à 3,5 ppm ($C\underline{H_2}S\rightarrow O$, AB, $J_{AB}$=17 Hz) - 8 H entre 1,3 et 2,3 ppm (cyclopentyl, M) - 9 H à 1,50 ppm ( 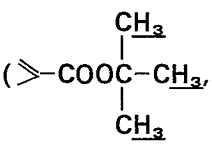 , S)

- 9 H à 1,35 ppm ( 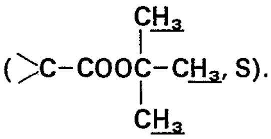 , S).

b) et c)

A partir du dérivé bromé ci-dessus et en opérant comme dans l'exemple 3 b) avec l'hydroxy-3 mercapto-5 triazine-1,2,4 et après déblocage des fonctions amine et acide selon l'exemple 3 c), on obtient le produit attendu.

*Exemples 6 à 8:*

En opérant comme indiqué dans les exemples 1, 3 et 4, à partir des dérivés bromés décrits dans les exemples 1 a), 3a) et 4a), mais en remplaçant l'hydroxy-3 mercapto-5 triazine-1,2,4 par une quantité équivalente d'hydroxy-5 mercapto-3 triazine-1,2,4, on obtient de la même façon, après déprotection:

— l'acide [(amino-2 thiazolyl-4)-2 méthoxyimino-2 acétamido]-7 [(hydroxy-5 triazin-1,2,4 yl-3)thiométhyl]-3 céphème-3 carboxylique-4 S-oxyde-1 isomère syn (CM 40 970);

— l'acide [(amino-2 thiazolyl-4)-2 (carboxy-2 propyl-2 oxyimino)-2 acétamido]-7 [(hydroxy-5 triazin-1,2,4 yl-3)thiométhyl]-3 céphème-3 carboxylique-4 S-oxyde-1 isomère syn (CM 40 766); et, respectivement,

— l'acide [(amino-2 thiazolyl-4)-2 (carboxy-1 cyclobutyl-1 oxyimino)-2 acétamido]-7 [(hydroxy-5 triazin-1,2,4 yl-3)thiométhyl]-3 céphème-3 carboxylique-4 S-oxyde-1 isomère syn (CM 40 881) dont les spectres de RMN sont indiqués dans le tableau ci-après.

| Exemple | Composé | Spectre de RMN |
|---|---|---|
| 6 | CM 40 970 | 1 H à 8,90 ppm (D, J=9 Hz, $CON\underline{H}$) - 1 H à 7,60 ppm (S, $\underline{H}$ triazine) - 1 H à 6,86 ppm (S, $\underline{H}$ thiazole) - 4 H entre 5,20 et 7,20 ppm (S. e., $N\underline{H_2}$, $O\underline{H}$, $COO\underline{H}$) - 1 H à 5,87 ppm (D de D, $J_1$=9 Hz, $J_2$=4 Hz, $\underline{H_7}$) - 1 H à 4,92 ppm (D, J=4 Hz, $\underline{H_6}$) - 1 H à 4,33 ppm (A de AB, $J_{AB}$=13 Hz, $C\underline{H_2}S$) - 1 H à 4,05 ppm (B de AB, $J_{AB}$=13 Hz, $C\underline{H_2}S$) - 5 H à 3,84 ppm (S. e., $C\underline{H_3}ON$, $C\underline{H_2}S\rightarrow O$) |
| 7 | CM 40 766 | 5 H entre 8,5 et 11 ppm (S. e., $NH_2$, OH, COOH) - 1 H à 8,45 ppm (D, J=9 Hz, $CON\underline{H}$) - 1 H à 7,62 ppm (S, H triazine) - 1 H à 6,89 ppm (S, H thiazole) - 1 H à 6,00 ppm (D de D, $J_1$=9 Hz, $J_2$=4 Hz, $H_7$) - 1 H à 4,95 ppm (D, J=4 Hz, $H_6$) - 1 H à 4,40 ppm (A de AB, $J_{AB}$=13 Hz, $C\underline{H_2}S$) - 1 H à 4,05 ppm (B de AB, $J_{AB}$=13 Hz, $C\underline{H_2}S$) - 2 H à 3,80 ppm (S. e., $C\underline{H_2}S\rightarrow O$) - 6 H à 1,46 ppm (S, 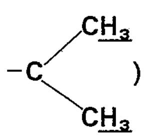 ) |
| 8 | CM 40 881 | 1 H à 8,70 ppm (D, J=9 Hz, $N\underline{H}CO$) - 4 H à 8,3 ppm (S. e., 2 $COO\underline{H}$, $N\underline{H_2}$) - 1 H à 7,63 ppm (S, H triazine) - 1 H à 6,89 ppm (S, H thiazole) - 1 H à 6,00 ppm (D de D, $J_1$=9 Hz, $J_2$=4 Hz, $H_7$) - 1 H à 5,00 ppm (D, J=4 Hz, $H_6$) - 1 H à 4,40 ppm (A de AB, $J_{AB}$=12 Hz, $C\underline{H_2}S$) - 1 H à 4,10 ppm (B de AB, $J_{AB}$=12 Hz, $C\underline{H_2}S$) - 2 H à 3,80 ppm (M, $CH_2S\rightarrow O$) - 6 H entre 1,5 et 2,5 ppm (M, 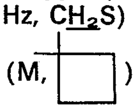 ) |

## Revendications

1. 3-(hydroxy-1,2,4-triazinyl)thiométhyl-céphalosporines de formule:

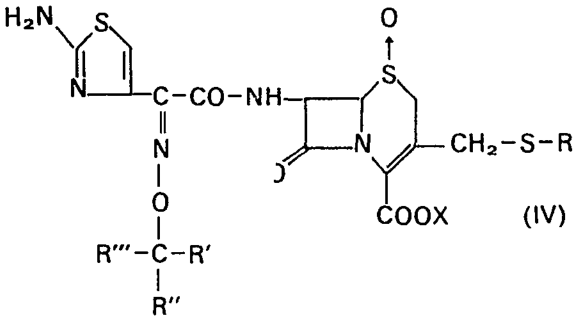

(IV)

dans laquelle:

— R représente un groupe 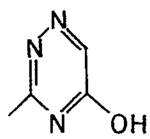

ou un groupe 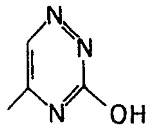

R' représente de l'hydrogène ou un groupe carboxyle COOX',

R'' et R''', qui peuvent être identiques ou différents, représentent chacun de l'hydrogène ou un groupe alkyle inférieur ayant de 1 à 3 atomes de carbone ou, ensemble, un radical de 1,3-propylène ou 1,4-butylène, X et X', qui peuvent être identiques ou différents, représentent de l'hydrogène ou un cation ou un ester ou hémiacétal facilement hydrolysable ou métaboliquement labile et pharmaceutiquement acceptable.

2. Médicaments actifs, notamment vis-à-vis des bactéries Gram négatives, caractérisés en ce qu'ils contiennent au moins une céphalosporine selon la revendication 1.

3. Médicaments selon la revendication 2, caractérisés en ce qu'ils contiennent par dose unitaire de 10 à 10000 mg de produit actif (céphalosporine).

4. Procédé pour la préparation des céphalosporines selon la revendication 1, caractérisé en ce que:

— on utilise comme produit de départ le composé de formule:

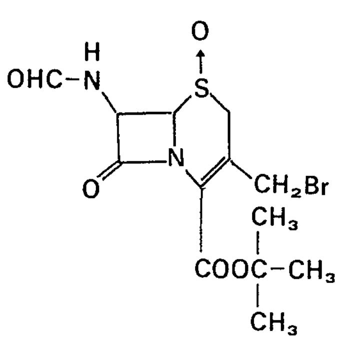

— on fait réagir ce produit avec un composé de formule R−SH, ladite réaction étant effectuée dans le diméthylformamide en présence d'une base comme la triéthylamine,

— on libère le groupe amino du produit obtenu par action du chlorure de thionyle en solution,

— on acyle le produit obtenu à l'aide d'un dérivé de formule

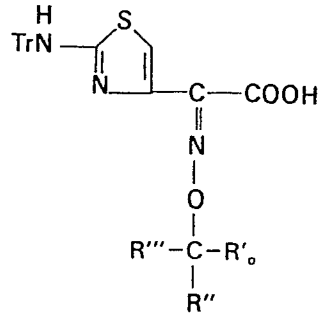

dans laquelle Tr est un radical protecteur du radical amino, comme par exemple un groupe trityle, et $R'_0$ est H ou un groupe $COOX'_0$ dans lequel $X'_0$ est lui-même un groupe hémiacétal ou ester facilement hydrolysable, le groupe carboxyle dudit dérivé ayant été activé par exemple par transformation en anhydride,

— on élimine de façon connue, de préférence par hydrolyse acide, le groupe protecteur (Tr), l'ester tertiobutylique et, éventuellement, $X'_0$ de façon à obtenir un produit de formule selon la revendication 1, dans lequel X est H,

— et on transforme éventuellement les produits obtenus de façon à substituer de façon connue ledit H par le substituant X.

## Patentansprüche

1. 3-(Hydroxy-1,2,4-triazinyl)-thiomethyl-cephalosporine der Formel

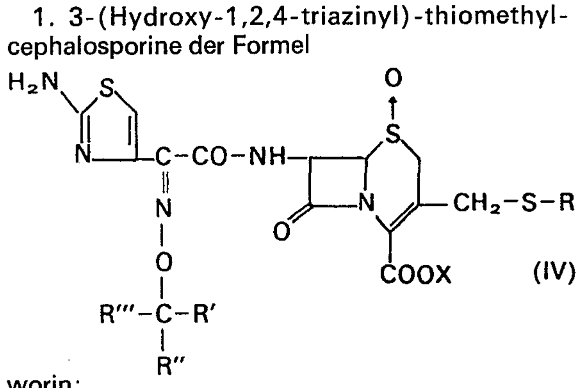

(IV)

worin:

— R eine Gruppe 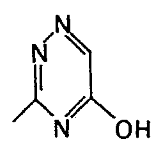

oder eine Gruppe 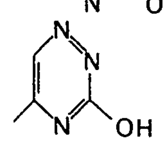

darstellt, R' für Wasserstoff oder eine Carboxylgruppe COOX' steht, R'' und R''', die gleich oder verschieden sein können, jeweils Wasserstoff oder eine Niedrigalkylgruppe mit 1 bis 3 Kohlenstoffatomen oder gemeinsam ein 1,3-Propylen- oder 1,4-Butylenradikal darstellen, X und X', die gleich oder verschieden sein können, Wasserstoff oder

ein Kation oder einen Ester oder ein Hemiacetal, die leicht hydrolysierbar oder metabolisch labil und pharmazeutisch akzeptabel sind, darstellen.

2. Medikamente, insbesondere wirksam gegen gramnegative Bakterien, dadurch gekennzeichnet, dass sie zumindest ein Cephalosporin nach Anspruch 1 enthalten.

3. Medikamente nach Anspruch 2, dadurch gekennzeichnet, dass sie pro Einheitsdosis 10 bis 10 000 mg Wirkstoff (Cephalosporin) enthalten.

4. Verfahren zur Herstellung der Cephalosporine nach Anspruch 1, dadurch gekennzeichnet, dass

— als Ausgangsprodukt die Verbindung der Formel:

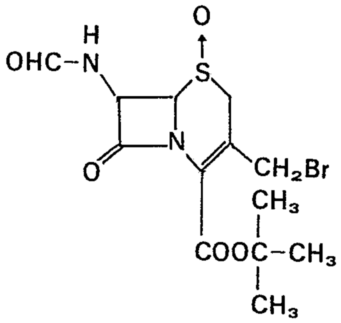

verwendet wird,

— dieses Produkt mit einer Verbindung der Formel R−SH reagieren lassen wird, wobei die Reaktion in Dimethylformamid in Gegenwart einer Base, wie Triäthylamin, durchgeführt wird,

— die Aminogruppe des erhaltenen Produkts durch Einwirken von in Lösung befindlichem Thionylchlorid freigesetzt wird,

— das erhaltene Produkt mittels eines Derivats der Formel

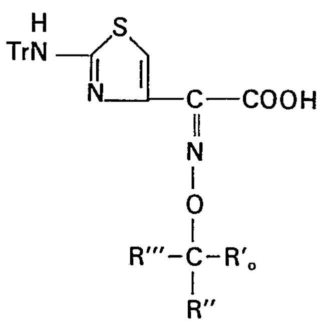

worin Tr eine Schutzgruppe des Aminoradikals, beispielsweise eine Tritylgruppe, ist und $R'_0$ H oder eine $COOX'_0$-Gruppe ist, worin $X'_0$ selbst eine Hemiacetal- oder Estergruppe darstellt, die leicht hydrolysierbar sind, acyliert wird, wobei die Carboxylgruppe des Derivats beispielsweise durch Umwandlung in ein Anhydrid aktiviert wurde,

— die Schutzgruppe (Tr), der tert. Butylester und gegebenenfalls $X'_0$ auf bekannte Weise, vorzugsweise durch saure Hydrolyse, entfernt werden, sodass ein Produkt der Formel gemäss Anspruch 1, worin X H bedeutet, erhalten wird,

— und gegebenenfalls die erhaltenen Produkte durch Substituieren des H durch den Substituenten X auf bekannte Weise umgewandelt werden.

**Claims**

1. 3-(hydroxy-1,2,4-triazinyl)thiomethylcephalosporins with formulae:

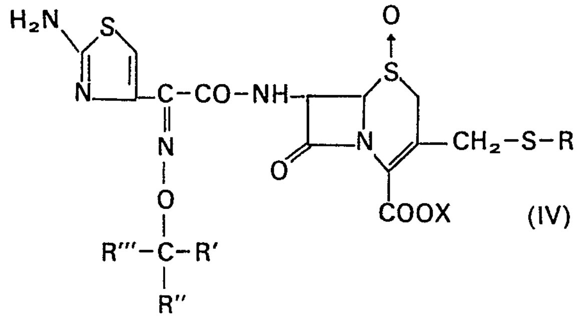

(IV)

in which:

— R represents a 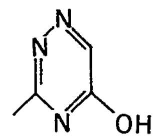 group

or a 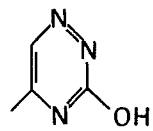

R′ is hydrogen or a COOX′ carboxyl group

R″ and R‴ can be identical or different, each representing hydrogen or a lower alkyl group with 1 to 3 carbon atoms or together, a 1,3-propylene or 1,4-butylene radical, X and X′ can be identical or different, represent hydrogen or a cation or an easily hydrolizable or metabolically labile and pharmaceutically acceptable ester of hemiacetal.

2. Drugs active in particular against gramnegative bacteria, characterized in that they comprise at least one cephalosporin according to claim 1.

3. Drugs according to claim 2, characterized in that they contain per unit dose from 10 to 10.000 mg of active product (cephalosporine).

4. Process for the preparation of cephalosporins according to claim 1, characterized in that

— a compound of formula

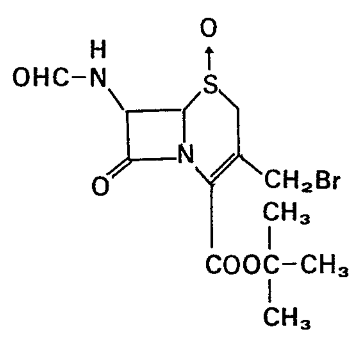

is used as starting product,

— said product is reacted with a compound of the formula R−SH, the reaction being carried out in dimethyl formamide in the presence of a base, such as triethylamine

— the amino group of the obtained product is liberated by treatment with thionyl chloride solution,

— the product obtained is then acylated using a derivative with the formula:

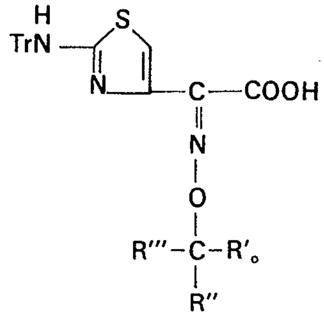

in which Tr is a radical which protects the amino radical, such as a trityl group and wherein $R'_0$ is H or a $COOX'_0$ wherein $X'_0$ is an easily hydrolizable hemiacetal or ester, the carboxyl group of the derivative being activated by transformation into an anhydride,

— the protective group (Tr), the tertiobutyl ester, and possibly $X'_0$ are eliminated, using a known method so as to obtain a product according to claim 1 where X is equivalent to H,

— these products can be transformed so as to substitute in known manner a different X substituent for the H.